# EUROPEAN PATENT SPECIFICATION

(11) **EP 0 423 855 B1**
(45) Date of publication and mention of the grant of the patent: **20.07.1994**
(21) Application number: 90202488.4
(22) Date of filing: 19.09.1990
(51) Int. Cl.: B65D 75/36, A61M 25/00

(54) **Packaging**
Verpackung
Emballage

(30) Priority: 16.10.1989 NL 8902563; 04.07.1990 NL 9001525
(43) Date of publication of application: 24.04.1991
(73) Proprietor: CORDIS EUROPA N.V., NL-9301 LJ Roden (NL)
(72) Inventor: Van Veen, Jimmy Eduard, NL-9300 XS Roden (NL); Kremer, Arjan, NL-9302 BH Roden (NL)
(74) Representative: 't Jong, Bastiaan Jacobus

(56) References cited:
- WO-A-89/04685
- US-A- 3 104 172
- US-A- 3 839 841
- US-A- 3 934 721
- US-A- 4 248 236

## Description

The invention relates to a packaging for at least one element with a thin elongate portion comprising at least two elongate flexible sheets adhered to each other which form between them a space for receiving said at least one element, at least one of said sheets being a blistersheet and said space being formed by a bulging-out portion corresponding with the form of said element in said sheet.

Such a packaging is known from US-A 3 104 172.

The invention has for its object to provide a packaging of this kind wherein an element with a thin elongate part can be packaged in an economical manner.

With the packaging according to the invention this is achieved in that the sheet in the part of the bulging-out portion corresponding with the thin elongate portion of said element has an at least partly corrugated profile. As a result of the corrugated profiling of the part of the bulging-out portion corresponding with the thin elongate portion of the element it is highly flexible without there being the risk of the material of the sheet buckling and tearing at the location of the bulging-out portion. With a packaging for a catheter buckling would mean the catheter becoming unusable and tearing the loss of the sterility thereof.

With the invention it has become possible to arrange in a suitable manner in a blister pack a thin elongate element or an element comprising a thin elongate portion.

An attendant advantage of the packaging according to the invention is that after use this can be easily crumpled up so that the packaging can be thrown away and disposed of without problems.

The invention also relates to and provides an assembly of packaging and a catheter packaged therein having an outer end curved in a particular shape, comprising means for confining said curved outer end. Such an assembly is known from WO 89/04685. When the packaged catheter has an outer end curved in a particular shape, as is usual with catheters for angio-graphic purposes, the packaging is preferably embodied as specified in claim 2. It is usual to curve the outer end of catheters into the desired shape when they are heated, whereupon the curved form is fixed therein as a result of cooling. After packaging of the catheter it has to be sterilized. In a certain phase of the sterilization process the assembly of packaging and catheter is heated. In the case of a packaging according to the state of the art the risk exists hereby that the curved outer end curves out slightly because of the raised temperature and thereby no longer maintains the required particular form. The confining means prevent this. With the assembly according to the invention as characterized in claim 2, the confining means are integrated in the packaging in an elegant way.

In the manufacture of catheters which have a connecting member at one end, variations in length occur through unavoidable manufacturing tolerances. In order to be able to arrange catheters in the packaging according to the invention in a properly fixed manner the step of claim 3 is preferably applied. The fixing means ensure that this connecting member can be arranged immovably in the packaging irrespective of the longitudinal position of the connecting member relative to the remaining portion of the catheter.

A favourable embodiment of the fixing means is characterized in claim 4.

According to a further development of the invention the fixing means are embodied as characterized in claim 5. The recess can receive the connecting member in a precisely fitting manner so that it does not become wedged in the packaging. The removal of the catheter from the packaging is hereby facilitated. Accordingly to a very favourable embodiment as claimed in claim 6, the surface of the first flexible sheet carries a layer of polyethylene on the side to be welded to the second sheet. In this way damage to the catheter upon removal of the catheter from the packaging can be prevented. Dammage in the form of one or more small flat areas on the point, which has the specific curved shape appeared on catheter removed from packagings not having this polyethylene layer. Although these flat areas were scarcely visible to the naked eye, they were nevertheless such that they would result during manufacture in rejection of the catheter. Surprisingly and unexpectedly it was found that the damage was the result of the end part of the catheter brushing along the inside of the packaging during withdrawal thereof immediately prior to use. In equally surprising manner it has been found that such "wear and tear" damage occurs during rubbing contact with very many plastics but not, however, with polyethylene. By thus using for the sheet a material that carries on the inside a layer of polyethylene, the damage is prevented and the high quality of a catheter packed with the packaging according to the invention remains preserved.

The second sheet of the packaging typically consists of a paper permeable to sterilizing gas which can be provided with a plastic layer. According to the invention a paper provided with a layer of polyethylene is preferably used for this second sheet.

A material suitable for the blister packaging for an angiographic catheter has a flexible sheet of PET-G, or glycol-modified polyethylene terephthalate, with a layer of polyethylene.

The invention is particularly favourable in use for a packaging as characterized in claim 8. Such a packaging is opened by pulling apart the sheets lying free of one another until the weld connection on the narrow side of the packaging is broken and an end of the catheter can be grasped.

The invention will be further elucidated in the following description with reference to the embodiments shown in the figures.
Fig. 1 shows a packaging according to the invention in partly broken away perspective view;
Fig. 2 shows a portion of the packaging from fig. 1 on a larger scale;
Fig. 3 and 4 show parts of the packaging from fig. 1 and 2 in curved position;
Fig. 5 shows an alternative embodiment of a portion of a packaging according to the invention;
Fig. 6 shows a partly sectional top view of an alternative embodiment;
Fig. 7 shows a view corresponding with fig. 5 of a portion of an alternative embodiment;
Fig. 8 shows a corresponding view of yet another embodiment.
Fig. 9 shows a packaging according to a preferred embodiment of the invention in a partly broken away perspective view corresponding to fig. 1;
Fig. 10 shows a section of the packaging along line X-X in fig. 9.

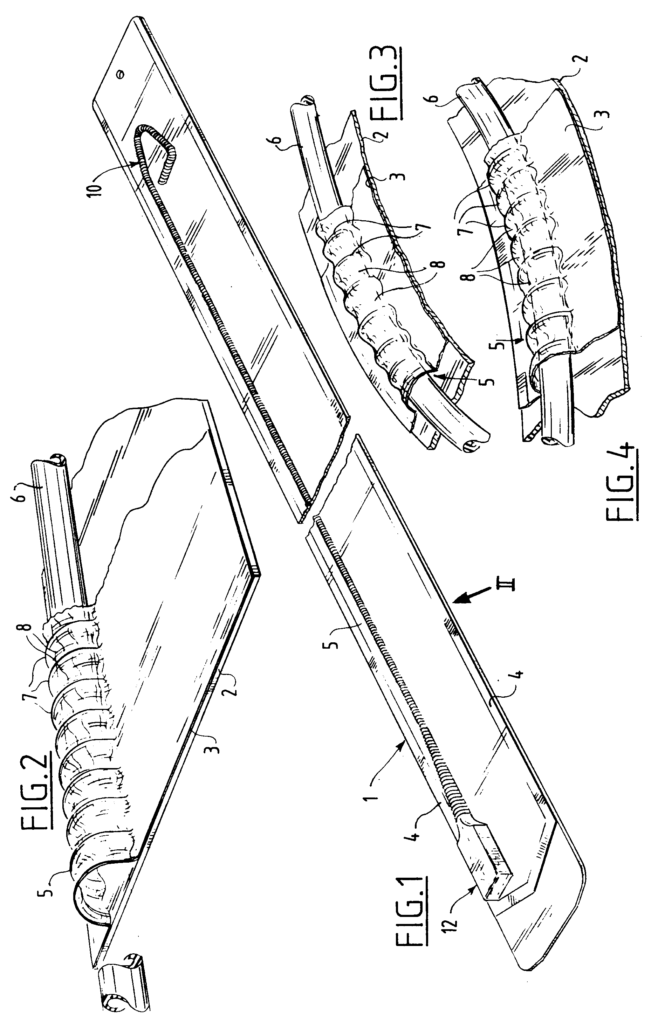

The packaging 1 shown in fig. 1 is intended for an angio-graphic catheter which is thin and elongate. The packaging 1 is embodied as a so-called blister pack and comprises a first sheet 2 and a second sheet 3 which is laid on the first sheet 2 and connected thereto along the edges 4 by a heat treatment. The upper sheet 3 consists preferably of a transparent plastic while the lower sheet 2 consists of plastic or plastic-coated paper or cardboard.

A bulging-out portion 5 is formed in the upper sheet 3 so that a space is formed between the sheets 2 and 3 at the location of the bulging-out portion, in which space the catheter 6 is received. As fig. 2 clearly shows the bulging-out portion 5 has a corrugated profile. Thus formed in the lengthwise direction of the bulging-out portion 5 are arcuate wave tops 7 and wave hollows 8. As a result of this corrugated profiling of the bulging-out portion 5 the sheet 3 can bend in a flexible manner without such great forces occurring in the bulging-out portion 5 that the material of the sheet 3 collapses.

Fig. 3 shows a portion of the packaging with the bulging-out portion 5 which is curved spherically while fig. 4 shows a corresponding portion which has hollow curves. In the situation in fig. 3 the waved profile in the upper portion of the bulging-out portion 5 is slightly stretched while in the situation of fig. 4 it is pressed slightly inward. With this deformation only relatively small bending loads occur in the material. If the bulging-out portions 5 were formed in the manner usual for blister packs, that is to say with a smooth, continuous wall without the corrugated profile, a high concentration of tension occurs very rapidly when bending takes place such that the material buckles and tears. Leaving aside the objection of the shabby appearance of a packaging with such buckles, the catheter arranged in such a packaging would also have become unusable because the sterility will no longer be ensured and the catheter will also be damaged.

The bulging-out portion 5 of the packaging shown in fig. 1 has a curved portion 10 which is intended for receiving an outer end of the catheter for packaging curved in a corresponding form. The end part 10 of the bulging-out portion is dimensioned and geared to the catheter for packaging such that in the packaged state the curved outer end of the catheter is locked against bending out of the particular desired form. During a sterilizing treatment carried out after the catheter has been packaged the assembly of packaging and catheter is subjected to an increased temperature, whereby the danger arises that the curved form of the outer end arranged by thermoplastic processing may to a greater or lesser extent be lost. However, because the curved outer end of the catheter remains enclosed in the bulging-out portion, the shape of the catheter is also preserved at high temperatures.

In the manufacture of long catheters variations in the total length of the catheter inevitably occur through manufacturing tolerances. The position of the connecting member may vary some millimetres relative to the curved end portion.

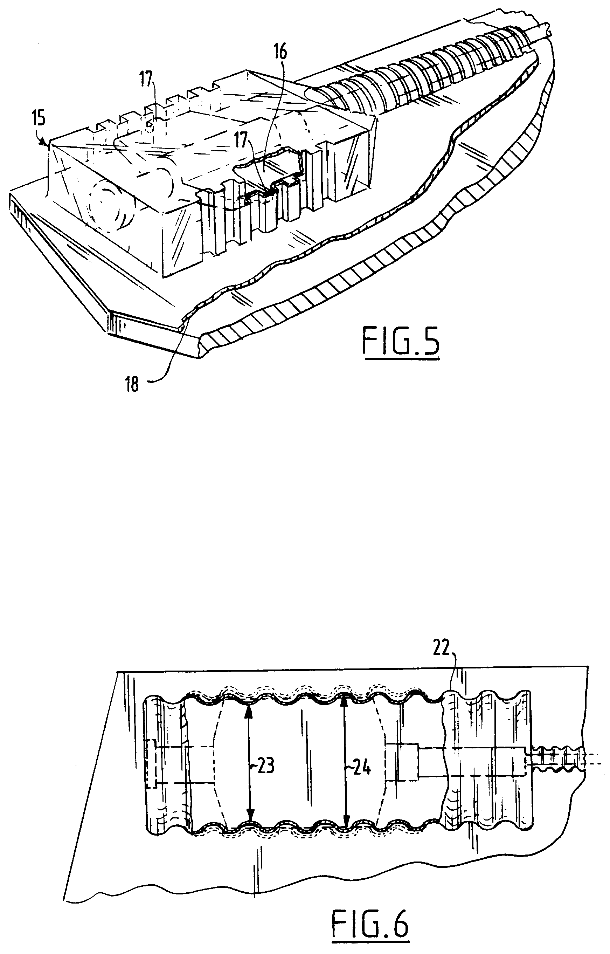

Fig. 5 shows a portion of a packaging according to the invention which can still fix the coupling member well despite such variations. As shown the upper sheet 18 in this embodiment comprises a larger bulging-out portion 15 at the position where the coupling member 16 of a catheter has to be arranged. This bulging-out portion 15 also has a corrugated profile. The coupling member 16 is provided on either side with projections 17 which can fall into oppositely situated wave tops of the bulging-out portion 15. The bulging-out portion 15 has a greater length than the coupling member 16 so that the coupling member can be arranged in the bulging-out portion 15 in a number of different positions, corresponding with the location of the wave tops. Through the engaging of the projections 17 in the profile of the bulging-out portion 15 the coupling member 16 and therefore the catheter is properly locked in lengthwise direction.

In the embodiment of fig. 6 the bulging-out portion 22 likewise has a corrugated profile. The distance in section between wave hollows facing each other, which distance is indicated with arrow 23, is made somewhat smaller that the width of the coupling member designated with dashed lines, while the distance between the wave tops as indicated with arrow 24 is greater than this width dimension of the coupling member. When the catheter is laid in the bulging-out portion of the packaging the coupling member is pressed into the bulging-out portion 22. This thereby deflects into the position indicated with broken lines. Through the waved shape of the bulging-out portion this deflection takes place without damage to the material. The coupling member can therefore be held in a variable lengthwise position in the bulging-out portion 22 through friction.

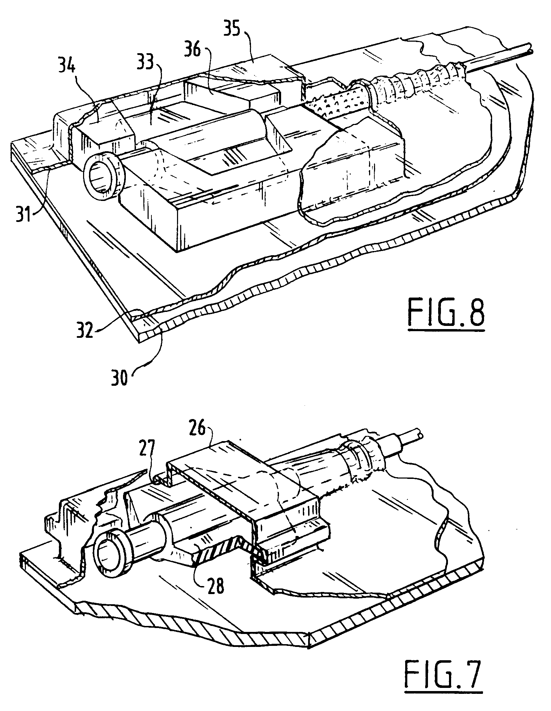

With the alternative embodiment in fig. 7 the bulging-out portion 26 is provided with recesses 27 which can receive the edges of the wings of the coupling member 28. The recesses 27 are dimensioned such that they can slidably accommodate the coupling member 28 with friction.

With the packaging in fig. 8 the coupling member 33 is arranged in a variable longitudinal position in the packaging without friction but in a close-fitting manner. Arranged between the lower sheet 30 and the upper sheet 31 is a sheet-like member 32 which is provided with a bulge 34 which defines a cavity 36 wherein the coupling member 33 is close fittingly arranged. The bulging-out portion 35 in the upper sheet 31 is formed such that the bulge 34 of the sheet-like member 32 can be arranged therein in a variable longitudinal position.

During packaging the catheter the coupling member is laid in the cavity 36 of the at that moment still loose sheet 32. The remaining part of the catheter is laid in the bulging-out portion of the upper sheet 31 whereby the bulge 34 falls into the longitudinal position in the bulging-out portion 35 determined by the specific length of the catheter. When the thus assembled packaging is then sealed, the lower sheet 30 and upper sheet 31 are welded together along their edges in the previously described manner and the holder sheet 32 is also welded in position. The sheet 32 is thus fixed relative to the upper sheet 31 and the lower sheet 30 and in this way the coupling member 33 is fixed in the lengthwise direction of the catheter.

The packaging is further embodied such that when the packaging is opened by pulling the sheets away from each other the sheet-like member 32 is taken away with the lower sheet so that the upper sheet 31 is therefore pulled loose from the sheet-like member 32 and the coupling member 33, as seen in fig. 8, can be released from the cavity 36 in upward direction. It is of course also possible to embody a sheet-like member such that the cavity for the coupling member is accessible from below and that when the packaging is pulled open the sheet-like member with the cavity is taken along by the upper sheet.

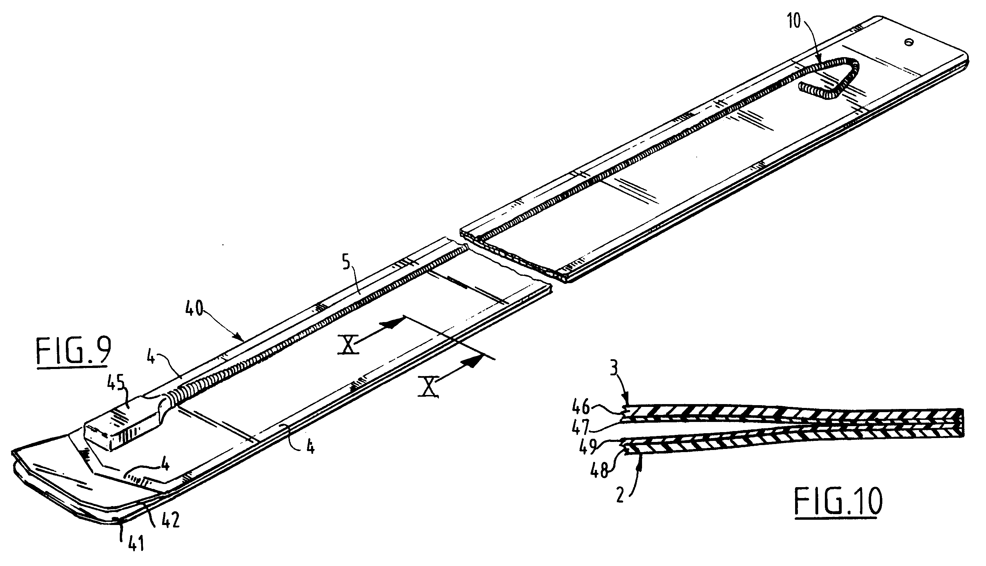

The blister packaging 40 shown in fig. 9 corresponds for a substantial part with the packaging 1 of fig. 1. Corresponding elements are referred to with the same reference numerals.

The weld connection 4 of the two sheets 2, 3 on the left-hand side of the packaging 1 in fig. 9 lies at an interval from the end so that two lips 41, 42 of the respective sheets 2, 3 are formed which are not joined to one another. In order to take the catheter 6 out of the packaging the lips 41 and 42 are gripped and pulled apart. The weld connection 4 close to the narrow end is thereby broken and the connecting part of the catheter, not further shown here, arranged in the widened part 15 of the pressed-out portion 5 can be grasped. The catheter is then taken out of the packaging by being pulled therefrom in lengthwise direction. The end part therein slides through between the sheets 2 and 3 and rubs along the mutually facing surfaces of these sheets.

As shown in fig. 10, the sheet 3, which substantially consists for example of glycol-modified polyethylene terephthalate (PET-G) 46, is preferably provided with a thin layer of polyethylene 47 on the side facing the second sheet 2. The second sheet 2, which substantially consists for example of paper 48, can likewise be covered with a layer of polyethylene 49. When the catheter 6 slides outward it is therefore continuously in contact with polyethylene, whereby the wear and tear phenomenon occurring in the case of contact with other plastics does not take place.

Since the paper of the second sheet 2 yields more easily that the plastic of the first sheet 3, the chance of damage through "wear and tear" during the rubbing along this sheet 2 is considerably smaller. Within the scope of the invention it is therefore not essential that the paper of the sheet 2 also carries a layer of polyethylene.

The material of the sheet 3 does not have to consist as shown of only two layers. Instead of for instance continuous PET-G a laminate can also be used. Another material can also be used instead of the paper 48.

The sheet of the packaging provided with the bulging-out portion can be formed in a per se known manner for blister packs by subjecting a thermoplastic material to a vacuum forming technique.

When a hermetic packaging is not necessary, as it is with catheters, the sheet of the packaging bearing the pressed-out portion can for example be of a suitable thin cardboard and the bulging-out portion with corrugated profile can be arranged therein by a pressing operation. The invention is not limited to the manufacture of the packaging with a determined design technique. All techniques which can arrange an elongate bulging-out portion having at least partly a corrugated profile in a flexible sheet are applicable for the invention.

In addition to use for elements which are themselves thin and elongate, such as catheters, the packaging according to the invention can also very well be used for elements of which only a portion is thin and elongate. At the location of the thin elongate portion the packaging is then embodied with a bulging-out portion having a corrugated profile.

## Claims

1. Packaging (1) for at least one element (6) with a thin elongate portion comprising at least two elongate flexible sheets (2, 3) adhered to each other which form between them a space for receiving said at least one element, at least one of said sheets (3) being a blistersheet and said space being formed by a bulging-out portion corresponding with the form of said element in said sheet, **characterized in that** the sheet (3) in the part of the bulging-out portion corresponding with the thin elongate portion of said element has an at least partly corrugated profile (7, 8).

2. Assembly of packaging (1) and a catheter (6) packaged therein having an outer end being curved, comprising means for confining said curved outer end, **characterized in that** the packaging is a packaging (1) according to claim 1, the part (10) of the bulging-out portion corresponding with said curved outer end being formed such that said curved outer end is confined therein and locked against curving out of said particular shape.

3. Assembly of packaging as claimed in claim 1 or 2 and a catheter packaged therein having a connecting member (16) at one end, **characterized in that** the part (12) of the bulging-out portion corresponding with said connecting member (16) comprises fixing means (17) for fixing said connecting member (16) in a variable longitudinal position.

4. Assembly as claimed in claim 3, **characterized in that** the fixing means (17) accommodate the connecting member (16) for sliding with friction.

5. Assembly as claimed in claim 3, **characterized in that** the fixing means comprise a separate sheet-like member (32) for connection to at least one of the sheets (30, 31) with a recess (36) receiving the connecting member (33) in a fixed position.

6. Assembly as claimed in any of the claims 2-5, **characterized in that** the surface of the first flexible sheet (3) carries a layer (47) of polyethylene on the side to be welded to the second sheet (2).

7. Assembly as claimed in claim 6, **characterized in that** the second sheet (2) consists of a paper sheet (48) provided with a layer of polyethylene (49).

8. Packaging or assembly as claimed in any of the preceding claims, **characterized in that** the weld connection of the two sheets on a narrow side of the packaging lies at an interval from the end in order to form an end region wherein the sheets are free of one another.

## Patentansprüche

1. Verpackung (1) für mindestens ein Element (6) mit einem dünnen länglichen Abschnitt, mit mindestens zwei länglichen flexiblen Blättern (2, 3), die aneinander haften, die einen Raum zwischen sich zum Aufnehmen des mindestens einen Elementes bilden, wobei mindestens eines der Blätter ein Blisterblatt (3) ist und der Raum durch einen ausgebeulten Abschnitt gebildet ist, der der Form des Elementes in dem Blatt entspricht,
dadurch gekennzeichnet, daß das Blatt (3) in dem Teil des ausgebeulten Abschnittes, der dem dünnen länglichen Abschnitt des Elementes entspricht, mindestens ein teilweise gewelltes Profil (7, 8) aufweist.

2. Anordnung einer Verpackung (1) und eines darin verpackten Katheters (6) mit einem äußeren Ende, das gekrümmt ist, mit Mitteln zum Einschließen des gekrümmten äußeren Endes,
dadurch gekennzeichnet, daß die Verpackung eine Verpackung (1) gemäß Anspruch 1 ist, der Teil (10) des ausgebeulten Abschnittes, der dem äußeren Ende entspricht, so gebildet ist, daß das gekrümmte äußere Ende darin eingeschlossen ist und gegen Verbiegen aus der speziellen Form verriegelt ist.

3. Anordnung einer Verpackung nach Anspruch 1 oder 2 und ein darin verpackter Katheter mit einem Verbindungsteil (16) an einem Ende,
dadurch gekennzeichnet, daß der Teil (12) des ausgebeulten Abschnittes, der dem Verbindungsteil (16) entspricht, Befestigungsmittel (17) zum Befestigen des Verbindungsteiles (16) an einer variablen Längsposition aufweist.

4. Anordnung nach Anspruch 3,
dadurch gekennzeichnet, daß das Befestigungsmittel (17) das Verbindungsteil (16) zum Gleiten mit Reibung aufnimmt.

5. Anordnung nach Anspruch 3,
dadurch gekennzeichnet, daß das Befestigungsmittel ein separates blattartiges Teil (32) zum Verbinden von mindestens einem der Blätter (30, 31) mit einer Ausnehmung (36) aufweist, die das Verbindungsteil (33) in einer festen Position empfängt.

6. Anordnung nach einem der Ansprüche 2 - 5,
dadurch gekennzeichnet, daß die Oberfläche des ersten flexiblen Blattes (3) eine Schicht (47) aus Polyäthylen auf der Seite trägt, die mit dem zweiten Blatt (2) zu verschweißen ist.

7. Anordnung nach Anspruch 6,
dadurch gekennzeichnet, daß das zweite Blatt (2) aus einem Papierblatt (48) besteht, das mit einer Schicht von Polyäthylen (49) versehen ist.

8. Verpackung oder Anordnung nach einem der vorhergehenden Ansprüche,
dadurch gekennzeichnet, daß die Schweißverbindung der zwei Blätter auf einer Schmalseite der Verpackung in einem Abstand von dem Ende liegt, damit ein Endbereich gebildet wird, in dem die Blätter frei voneinander sind.

## Revendications

1. Emballage (1) destiné à au moins un élément (6) présentant une fine partie allongée, comprenant au moins deux feuilles souples allongées (2, 3) collées l'une à l'autre qui forment, entre elles, un espace destiné à loger l'élément, au nombre minimum d'un, au moins l'une (3) des feuilles étant un blistère et l'espace étant formé par une partie renflée dans la feuille correspondant à la forme de l'élément, caractérisé en ce que la feuille (3), dans la zone de la partie renflée correspondant à la fine partie allongée de l'élément, présente un profit (7, 8) au moins partiellement ondulé.

2. Ensemble constitué de l'emballage (1) et d'un cathéter (6) emballé dans celui-ci, présentant une extrémité extérieure recourbée , comprenant des moyens pour retenir cette extrémité extérieure recourbée, caractérisé en ce que l'emballage est un emballage selon la revendication 1, la partie (10) du renflement correspondant à l'extrémité extérieure recourbée étant formée de telle sorte que l'extrémité extérieure recourbée y soit retenue et qu'elle soit empêchée de quitter cette forme courbe particulière.

3. Ensemble constitué de |'emballage selon la revendication 1 ou 2 et d'un cathéter emballé dans celui-ci, présentant un élément de connexion (16) à une extrémité, caractérisé en ce que la partie (12) du renflement correspondant à cet élément de connexion (16) comprend des moyens de fixation (17) destinés à fixer l'élément de connexion (16) dans une position longitudinale variable.

4. Ensemble selon la revendication 3, caractérisé en ce que les moyens de fixation (17) logent l'élément de connexion (16) de telle sorte qu'il puisse coulisser avec friction.

5. Ensemble selon la revendication 3, caractérisé en ce que les moyens de fixation comprennent un élément (32) séparé semblable à une feuille, destiné à être relié à au moins l'une des feuilles (30, 31), avec un renfoncement (36) logeant l'élément de connexion (33) dans une position fixe.

6. Ensemble selon l'une quelconque des revendications 2 à 5, caractérisé en ce que la surface de la première feuille souple (3) porte une couche (47) de polyéthylène, du côté destiné à être soudé à la deuxième feuille (2).

7. Ensemble selon la revendication 6, caractérisé en ce que la deuxième feuille (2) est constituée d'une feuille de papier (48) revêtue d'une couche de polyéthylène (49).

8. Emballage ou ensemble selon l'une quelconque des revendications précédentes, caractérisé en ce que la soudure entre les deux feuilles, sur un chant de l'emballage, est située à un certain intervalle de l'extrémité, afin de constituer une zone terminale dans laquelle les feuilles ne sont pas soudées.
